# EUROPÄISCHE PATENTANMELDUNG

(11) **EP 4 678 755 A1**
(43) Veröffentlichungstag der Anmeldung: **14.01.2026**
(21) Anmeldenummer: 24188316.4
(22) Anmeldetag: 12.07.2024
(51) Int. Cl.: C12P 7/18, C12N 9/04, C12P 19/02

(54) **VERFAHREN ZUR HERSTELLUNG VON EINER WÄSSERIGEN LÖSUNG ENTHALTEND D-MANNITOL**

(71) Anmelder: Annikki GmbH, 8074 Raaba-Grambach (AT)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend D-Mannitol, indem D-Fructose, welche in einer wässerigen Lösung gelöst vorliegt, mit einer NAD(P)H-abhängigen Oxidoreduktase unter Bildung von oxidiertem Kofaktor NAD(P)+ zu D-Mannitol reduziert wird, dadurch gekennzeichnet, dass der durch die Reduktion entstandene, oxidierte Kofaktor NAD(P)+ mit einer Alkoholdehydrogenase und einem sekundären Alkohol unter Bildung eines Ketons zu NAD(P)H reduziert wird. Die NAD(P)H-abhängige Oxidoreduktase ist Mannitol-Dehydrogenase I (EC 1.1.1.67, NADH-abhängig) oder Mannitol-Dehydrogenase II (EC 1.1.1.138, NADPH-abhängig).

## Beschreibung

Die vorliegende Erfindung betrifft ein enzymatisches Verfahren zur Herstellung einer wässerigen Lösung enthaltend D-Mannitol.

### Hintergrund der Erfindung

D-Mannitol ist ein sechswertiger Zuckeralkohol, der Anwendungen als nicht-kariogenes und kalorienarmes Süßungsmittel (für Diabetiker-Nahrung), Pharmazeutikum (Diuretikum, zur Behandlung von Nierenversagen oder als Hilfsstoff in Tabletten und Inhalern) für die Produktion von Spezialchemikalien hat (Saha et al., 2011; Bhatt et al., 2013; Chen et al., 2020).

Da die natürlichen Vorkommen (beispielsweise in Früchten und Gemüse, aber auch in Meeresalgen) den steigenden Bedarf an D-Mannitol nicht decken können (Saha et al., 2011), wurden und werden Verfahren zur synthetischen Herstellung von D-Mannitol entwickelt.

Mehr als 50 000 Tonnen D-Mannitol pro Jahr werden durch Hydrierung eines Sirups bestehend aus 50% D-Fructose sowie 50% D-Glucose bei hohen Drücken und Temperaturen an einem Raney-NickelKatalysator hergestellt. Das erhaltene Produkt ist eine Mischung der beiden Diastereomere D-Mannitol und D-Sorbitol (25:75), da nur die Hälfte der D-Fructose zu D-Mannitol umgewandelt wird. Zur Trennung der beiden Zuckeralkohole sind aufwendige und teure Verfahren notwendig (Bhatt et al., 2013).

Eine Alternative zur katalytischen Hydrierung sind biokatalytische Verfahren wie Fermentationen.

Generell sind Mikroorganismen wie Hefen, Fadenpilze sowie homo- und heterofermentative Milchsäurebakterien in der Lage, D-Mannitol aus verschiedenen Substraten wie D-Glucose, D-Fructose, Saccharose oder Glycerin fermentativ herzustellen. Bei den homofermentativen Milchsäurebakterien wird D-Mannitol über D-Mannitol-1-phosphat aus D-Fructose-6-phosphat hergestellt, welches wiederum aus D-Fructose und D-Glucose erhalten werden kann. Bei den heterofermentativen Milchsäurebakterien wird D-Fructose direkt zu D-Mannitol reduziert (mittels einer Mannitol-Dehydrogenase), während hingegen die D-Glucose nur als Kohlenstoffquelle für das Wachstum dient. Aufgrund ihres vorteilhaften Stoffwechsels werden die heterofermentativen Milchsäurebakterien wie *Lactobacillus* oder *Leuconostoc* bevorzugt zur fermentativen D-Mannitol-Produktion eingesetzt (Martínez-Miranda et al., 2022).

US 7358072 B2 beschreibt eine Fermentation im kontinuierlichen Fed-Batch-Verfahren mittels *Lactobacillus intermedius* NRRL B-3693, mit der aus D-Fructose (insgesamt 295 g) und D-Glucose (insgesamt 140 g, als sekundäre Kohlenstoffquelle) in 22 h 166 g/l D-Mannitol (was bei einem berechneten Volumen von 1,4 Liter 232 g D-Mannitol ergibt) produziert werden konnte. Insgesamt wurden nur ca. 53% der zugeführten Zucker (D-Fructose/D-Glucose) zu D-Mannitol umgesetzt.

Zhang et al. (2017) beschreiben eine Fermentation mittels *Leuconostoc pseudomesenteroides* CTCC G123, wobei als Kohlenstoffquelle Inulin aus den Wurzeln der Gemeinen Wegwarte diente. So konnte 76,4 g/l D-Mannitol aus 91,0 g/l D-Fructose erhalten werden, wobei noch zusätzlich 25,1 g/l D-Glucose benötigt wurde, die vom zellulären Stoffwechsel zu Nebenprodukten wie Lactat und Acetat umgesetzt wurde. Die Mannitol-Ausbeute betrug 0,84 g/g D-Fructose oder 0,66 g/g (D-Fructose + D-Glucose). Durch Modulation des Redox-Stoffwechsels der Zellen mittels Zugabe von Nicotinsäure (50 mg/l) konnte die finale Konzentration an D-Mannitol auf 88,1 g/l und die Ausbeute auf 0,94 g/g D-Fructose gesteigert werden.

Die hier beschriebenen fermentativen Verfahren (mit heterofermentativen Milchsäurebakterien) weisen einige Nachteile auf: 1. ineffiziente Umsetzung der eingesetzten Zucker, 2. Bildung von Nebenprodukten wie Lactat oder Acetat, 3. Bedarf an komplexen und teuren Stickstoffquellen (Hefe, Pepton oder Fleischextrakte) sowie 4. lange Fermentationszeiten, vor allem zum Erreichen von höheren D-Mannitol-Titern (Martínez-Miranda et al., 2022).

Diese Nachteile können mit zellfreien Verfahren (*in vitro*) umgangen werden, wobei in diesen die Reduktion von D-Fructose zu D-Mannitol mit einer Mannitol-Dehydrogenase (MDH) bewerkstelligt wird, welche als Kofaktor entweder Nicotinamidadenindinukleotid (NAD) (EC 1.1.1.67) oder Nicotinamidadenindinukleotidphosphat (NADP) (EC 1.1.1.138) benötigt (Martínez-Miranda et al., 2022).

US 7867740 B2 beschreibt eine thermostabile Mannitol-Dehydrogenase aus *Thermotoga maritima,* die in immobilisierter Form zusammen mit einer (ebenfalls thermostabilen) Glucose-Isomerase eingesetzt wurde, um in einem elektrochemischen Reaktorsystem D-Glucose zu D-Mannitol bei 60 °C umzusetzen.

Weitere Mannitol-Dehydrogenasen sind aus *Pseudomonas fluorescens* (Kavanagh et al., 2002) oder aus *Aspergillus fumigatus* (Krahulec et al., 2011) bekannt.

Um die Reduktion von D-Fructose zu D-Mannitol mit signifikanten Produktausbeuten zu ermöglichen, muss der hierfür benötigte Kofaktor NAD(P)H in einer thermodynamisch begünstigten Reaktion regeneriert werden.

Ein häufig eingesetztes enzymatisches Kofaktor-Regenerationssystem ist die Glucose-Dehydrogenase (GDH) mit D-Glucose als Substrat, welches mittels NAD(P)⁺ zu D-Gluconolacton oxidiert wird, wobei NAD(P)H gebildet wird. Das Lacton hydrolysiert im wässerigen Milieu zu D-Gluconsäure/D-Gluconat.

Kulbe et al. (1987) koppelten eine NADH-abhängige Mannitol-Dehydrogenase aus *Saccharomyces cerevisiae* mit einer NAD⁺-abhängigen Glucose-Dehydrogenase (GDH) aus *Bacillus megaterium,* um eine Mischung aus D-Fructose und D-Glucose zu D-Mannitol und D-Gluconat umzusetzen.

Alternativ zur GDH kann auch eine Formiat-Dehydrogenase (FDH), welche Formiat und NAD⁺ zu CO₂ und NADH umsetzt, zur Kofaktor-Regeneration eingesetzt werden. So wurde eine Kombination aus FDH und einer rekombinanten MDH aus *Pseudomonasfluorescens* DSM 50106 von Slatner et al. (1998) zur Umsetzung von D-Fructose (80% Umsatz) zu 72 g/l D-Mannitol verwendet. Ein ähnliches System bestehend aus einer rekombinanten MDH aus dem theromotoleranten Bakterium *Caldicellulosiruptor morganii* Rt8.B8 und einer FDH aus *Ogataea parapolymorpha* wurde von Xu et al. (2020) eingesetzt, um 400 mM (72,1 g/l) D-Fructose in 50 h zu 80% zu D-Mannitol zu reduzieren.

Weder die Kofaktor-Regeneration mittels GDH noch mittels FDH sind für großtechnische zellfreie Produktionsverfahren von D-Mannitol geeignet, da sie zum Teil gravierende Nachteile aufweisen.

Bei Verwendung der GDH fallen große Mengen wasserlösliches D-Gluconat an, welches abgetrennt werden muss. Darüber hinaus werden aufgrund der Bildung von D-Gluconsäure äquimolare Mengen an Base (z.B. NaOH) benötigt, um den pH-Wert der Reaktion konstant zu halten.

Die Verwendung einer FDH zur Kofaktor-Regeneration zeichnet sich durch folgende Nachteile aus: 1. Bildung von klimaschädlichem CO₂ als Oxidationsprodukt von Formiat, 2. Verschiebung des pH-Werts der Reaktion in den basischen Bereich (Neuhauser et al., 1998; Kratzer et al., 2015), 3. Produktion großer Mengen an Abfall bestehend aus nicht umgesetztem Natrium-Formiat und Natriumsulfat (bei Verwendung von Schwefelsäure zur pH-Kontrolle) sowie 4. die geringe spezifische Aktivität von Formiat-Dehydrogenasen (Boldt & Ansorge-Schumacher, 2020; Tishkov & Popov, 2004).

In einem Übersichtsartikel schrieben Bhatt et al. im Jahr 2013, dass die Reduktion von D-Fructose zu D-Mannitol mittels Mannitol-Dehydrogenase technisch nicht angewendet wird, da geeignete Kofaktor-Regenerationssysteme für NAD(P)H fehlen (Bhatt et al., 2013).

Erschwerend kommt hinzu, dass Mannitol-Dehydrogenasen wie z.B. aus *Saccharomyces cerevisiae* (Kulbe et al., 1986), *Candida magnoliae* (Lee et al., 2003) oder *Tuber borchii* (Ceccaroli et al., 2007) im Allgemeinen einer signifikanten Produktinhibierung (Soetaert et al., 1999) unterliegen, welche die Reduktion von D-Fructose zu D-Mannitol ökonomisch unattraktiv machen.

Nicht nur die Produktinhibierung, sondern auch die Inhibierung von Mannitol-Dehydrogenasen durch andere organische Verbindungen sind beschrieben. In Konferenzbeiträgen von Cosse et al. (2021) und Cosse et al. (2022) wurde ein Verfahren zur Oxidation von Ethanol zu Acetaldehyd mittels Alkoholdehydrogenase vorgestellt, bei dem zur Regeneration von NAD⁺ eine MDH aus *Pseudomonas fluorescens* (Wildtyp und N191D-Mutante) eingesetzt wurde. Für die verwendete MDH wurde die Inhibierung durch Ethanol und Acetaldehyd beobachtet (Cosse et al., 2021) - dies deckt sich mit früheren Berichten über die Inhibierung einer anderen MDH (aus *Candida magnoliae*) durch Ethanol (Lee et al., 2008; Bhatt et al., 2013). Die Acetaldehyd- sowie die D-Mannitol-Ausbeuten des Verfahrens sind nicht beschrieben (Cosse et al., 2021; Cosse et al., 2022).

Hier setzt nun die vorliegende Erfindung an und möchte ein enzymatisches Verfahren zur Herstellung von einer wässerigen Lösung enthaltend D-Mannitol zur Verfügung stellen, welches die zuvor beschriebenen Nachteile nicht aufweist und somit hohe Ausbeuten als z.B. Xu et al. (2020) und Slatner et al. (1998) ermöglicht.

### Detaillierte Beschreibung der Erfindung

Die Aufgabe zur Herstellung einer wässerigen Lösung enthaltend D-Mannitol wird erfindungsgemäß gelöst, indem D-Fructose, welche in einer wässerigen Lösung gelöst vorliegt, mit einer NAD(P)H-abhängigen Oxidoreduktase unter Bildung von oxidiertem Kofaktor NAD(P)⁺ zu D-Mannitol reduziert wird, dadurch gekennzeichnet, dass der durch die Reduktion entstandene, oxidierte Kofaktor NAD(P)⁺ mit einer Alkoholdehydrogenase und einem sekundären Alkohol unter Bildung eines Ketons zu NAD(P)H reduziert wird.

Bei dem sekundären Alkohol handelt es sich bevorzugt um 2-Propanol (Isopropanol), welches zu Aceton oxidiert wird.

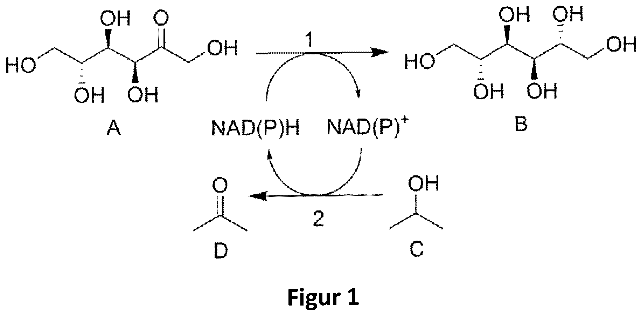

Das erfindungsgemäße Verfahren ist in der beiliegenden Figur 1 schematisch dargestellt, wobei die Bezeichnung A für D-Fructose, B für D-Mannitol, C für 2-Propanol und D für Aceton, 1 für Mannitol-Dehydrogenase und 2 für Alkoholdehydrogenase steht.

In einer bevorzugten Variante des erfindungsgemäßen Verfahrens wird die Reduktion von D-Fructose zu D-Mannitol *in vitro* durchgeführt.

Bei der NAD(P)H-abhängigen Oxidoreduktase handelt es sich bevorzugt um eine Mannitol-Dehydrogenase (NADH-abhängig: EC 1.1.1.67; NADPH-abhängig: EC 1.1.1.138) oder eine Sorbose-Reduktase (EC 1.1.1.289), wobei die Mannitol-Dehydrogenase besonders bevorzugt ist.

Der Einsatz von Alkoholdehydrogenasen zur Kofaktor-Regeneration in Kombination mit 2-Propanol als Kosubstrat ist bereits in US 10113192 B2, US 9644227 B2, US 10253340 B2 sowie von Xu et al. (2021) beschrieben.

Es hat sich überraschenderweise gezeigt, dass die Anwesenheit von 2-Propanol und Aceton zu keiner nennenswerten Inhibierung der Mannitol-Dehydrogenase führt. Diese Beobachtung steht im Kontrast zu Berichten aus der Literatur, dass der einfachere Alkohol Ethanol inhibierend auf die MDH aus *Pseudomonas fluorescens* (Cosse et al., 2021) und *Candida magnoliae* (Lee et al., 2008; Bhatt et al., 2013) wirkt. Darüber hinaus zeichnet sich das oxidierte Kosubstrat Aceton im Vergleich zu Acetaldehyd durch eine geringere Reaktivität und Giftigkeit aus.

Die besonders bevorzugte Konzentration von D-Fructose liegt zwischen 50 und 250 g/l.

Der besonders bevorzugte Temperaturbereich für das erfindungsgemäße Verfahren liegt zwischen 20 und 40 °C.

Der besonders bevorzugte pH-Bereich liegt zwischen 7,0 und 9,0.

Festes D-Mannitol kann beispielsweise durch Kristallisation oder mittels Sprühtrocknung aus der wässerigen Lösung gewonnen werden. Eine chromatographische Abtrennung von Nebenprodukten oder des Substrats ist nicht notwendig.

Die Abtrennung der Enzyme kann z.B. durch Zentrifugieren oder Ultrafiltration bewerkstelligt werden.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

Die Oxidoreduktase zur Reduktion von D-Fructose zu D-Mannitol ist eine Mannitol-Dehydrogenase und umfasst vorzugsweise eine Aminosäuresequenz, welche ausgewählt ist aus der Gruppe bestehend aus:
i) einer Aminosäuresequenz, die eine Identität zu SEQ ID Nr. 2 oder 4 von mindestens 80% aufweist,
ii) einer Aminosäuresequenz, die durch eine Nukleinsäure kodiert wird, die eine Identität zu SEQ ID Nr. 1 oder 3 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls bzw. an ein Nukleinsäuremolekül mit der Nukleinsäuresequenz SEQ ID Nr. 1 oder 3 bindet, wobei stringente Bedingungen vorzugsweise einen oder mehrere Waschschritte bei 65 °C und einer Salzkonzentration von 0,1x bis 2x SSC umfassen.

SEQ ID Nr. 1: 
ATGGAAGCATTGGTACTAACAGGCATCAAATCATTAGAGTTACAAGATCTACAACAACCAGAAGTAA
AACCAAACGAGGTTAAAATCCATACAGCGTTTGCAGGTATCTGTGGCACAGATCATGCTTTGTATGCT
GGTTTGCCTGGTTCAGCACCTGCTGTACCCCTATTGTTTTGGGACACGAAAATTCAGGTGTTGTTGCT
GAAATCGGATCAGCAGTTACAAATGTTAAAGTTGGTGATCGCGTCACAGTAGACCCTAACATTTATTG
TGGACAATGCAAATTCTGCCGTACAGGACGTCCAGAATTGTGTGAAAACTTATCAGCTGTTGGTGTGA
CACGTGATGGTGGTTTTGAAGAATACTTTACAGCACCAGCCTCAGTTGTTTATCCAATTCCTGATAATG
TATCATTAAAGTCAGCAGCTGTTGTTGAACCTATCTCATGTGCTGTTCACGGTATTCAATTGCTCAAAG
TAACACCTTACCAAAAAGCTTTGGTTATTGGTGACGGTTTCATGGGCGAATTATTTGTTCAACTTTTGC
AAGCATACGGTATTCACCAAGTTGATTTGGCTGGTATTGTTGATGAAAAGCTTGCGATGAACAAAGA
AAAGTTCGGGGCTAAAAACATATATAACACAATGAAGGGTGACAAGATTCCTGACGCAGAATATGAT
GTTGTCATTGAAGCTGTTGGTATGCCACAAACACAGGAAGCCGCTATTGAAGCAGCCGCTCGTGGTG
GACAAGTTTTGATGTTTGGTGTTGGTGGACCTGATGCTAAATTTGAAATGAATACTTATGAAATTTTCC
AAAAGCAATTGACAATTCAAGGATCATTTATCAATCCAAATGCGTTTGAAGATTCATTAGCTTTGCTAT
CATCTGGAAAATTGGATGTAGAATCTCTTATGTCTCATGAATTAGATTACAAGACTGTTGATGACTTCG
TGAATGGTAAGCTTGGCGTTGTTTCAAAAGCTGTTGTGAAGGTTGGCGGAGAAGAGGCATAA
SEQ ID Nr. 2: 
MILTSQTLSHLPDSILTSSYDRDAVTPGIVHLSVGNFHRAHQAVYTDRALALAGQSGWGIVGMGLMDHP
AEVAKAEALHAQNGLYTLRECPPNRPDTVRVVKSLVEYIHAPTDRDAALKRLTDPAIRIVSMTVTEGGYYM
DEAGRFMTEHPAIAEDLHRPVPHTAFGLMTEALRQRRDAGVAPFTILSCDNVPANGDVARNAVLSFARL
RDAELAEWIEANVAFPSCMVDRITPAVHPADVARLDAQSGIEDRVPVFCEDFIQWVVEDHFPAGRPAW
EQVGVLFTDDVEPYEQVKLRMLNASHSTLALPGVLMGYRLVDEAMGDEHLAALLEQFLRHDVEPQLTAP
PGISLPDYAAQLLQRFRNHAVSDQLLRITSDSTSKLPVFLRATAEDILKKAQDPRRIAFVLACYCAYLTGRDD
VEATFAVTEPRLSAEDRALALDANPAKALEMSVFAGWGLENSAEFLTRFIQTREDLKTRGTAAVLKDLVAN
V
SEQ ID Nr. 3: 
ATGTCACACCCAACATCAGTTATTAACGAGCAGGTAGGCCCATTACCAACTAAGGCCCACAACTTTC
TAAGAATGTGAATGATTTGTTCTCATTAAAGGGTAAGGTAGCTTCTGTCACCGGTTCCTCAGGAGGGA
TTGGTTGGGCTGTAGCTGAAGCTTATGCCCAAGCAGGAGCAGACGTTGCTGTTTGGTACAATTCTAA
AAATGCAGATGCTAAGGCTGAATACTTAACTAAGACTTACGGTGTGAAGTCGAAGGCATACAAGTGT
AACATTTCTGATCCAGAAGACGTCGAAAAGGTAATTGGACAAATTGAAAAGGATTTCGGTACCATTG
ACGTTTTCGTTGCCAATGCCGGTGTTCCATGGACAGAAGGTAGAAGTATTGAAGTTGAAGGATATGA
TTCGTGGAAGAAGGTTATAGATTTGGACTTGAGTGGTGTCTACTATTGCGCTAAGGCTGTCGGAAAG
ATTTTCAAGAAGAATGGTAAGGGTTCGCTTGTGTTCACAGCATCAATGTCTGGCCACATTGTGAATGT
TCCACAATTGCAAGCTCCATACAACGCCGCAAAGGCCGGTGTTTTGCACTTGAGTAAGTCATTAGCTG
TCGAATGGGCCCCATTCGCTAGAGTTAACACCATTTCACCTGGTTATATTGCCACAGAAATTTCCGACT
TCGTTCCGGACGATGTCAAGGCTAAGTGGTGGCAATTAATTCCATTAGGAAGAGAAGCTCTTCCACA
AGAATTAGTTGGTGCTTACTTATATTTTGCATCCGATGCGTCTACTTACACTACAGGATCGGACTTATT
AGTCGATGGTGGTTACTCTGCTCCATAA
SEQ ID Nr. 4:

MSHPTSVINEQVGPLPTKAPQLSKNVNDLFSLKGKVASVTGSSGGIGWAVAEAYAQAGADVAVWYNSK
NADAKAEYLTKTYGVKSKAYKCNISDPEDVEKVIGQIEKDFGTIDVFVANAGVPWTEGRSIEVEGYDSWKK
VIDLDLSGVYYCAKAVGKIFKKNGKGSLVFTASMSGHIVNVPQLQAPYNAAKAGVLHLSKSLAVEWAPFA
RVNTISPGYIATEISDFVPDDVKAKWWQLIPLGREALPQELVGAYLYFASDASTYTTGSDLLVDGGYSAP

Die Oxidoreduktase zur Reduktion von D-Fructose zu D-Mannitol umfasst oder besteht vorzugsweise aus einer Aminosäuresequenz, die eine Identität zu SEQ ID Nr. 2 oder 4 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Reduktion von D-Fructose zu D-Mannitol die Aminosäuresequenz SEQ ID Nr. 2 oder 4 oder besteht aus dieser.

Alternativ umfasst oder besteht die Oxidoreduktase zur Reduktion von D-Fructose zu D-Mannitol aus D-Fructose vorzugsweise aus einer Aminosäuresequenz, die durch eine Nukleinsäure kodiert wird, die eine Identität zu SEQ ID Nr. 1 oder 3 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Reduktion von D-Fructose zu D-Mannitol kodiert, die Nukleinsäuresequenz SEQ ID Nr. 1 oder 3 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990), verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 28, eine Erwartung (E) von 0,05, M = 1, N = -2 und einen Vergleich beider Stränge. Für Aminosäuresequenzen verwendet das blastp-Programm als Vorgaben eine Wortlänge von 3 und eine Erwartung (E) von 0,05 und die BLOSUM62-Scoring-Matrix (Henikoff & Henikoff, 1989), Alignments (B) von 50, Erwartung (E) von 0,05, M = 1, N =-2.

Alternativ umfasst die Oxidoreduktase zur Reduktion von D-Fructose zu D-Mannitol vorzugsweise eine Aminosäuresequenz oder besteht aus dieser, die durch eine Nukleinsäure kodiert wird, die unter stringenten Bedingungen an einen komplementären Strang eines Nukleinsäuremoleküls bzw. an ein Nukleinsäuremolekül mit der Nukleinsäuresequenz SEQ ID Nr. 1 oder 3 bindet. Wie hierin verwendet, beziehen sich die stringenten Bedingungen auf Bedingungen, unter denen sogenannte spezifische Hybride, jedoch keine unspezifischen Hybride gebildet werden.

Beispielsweise kann die Hybridisierung durch herkömmlich bekannte Verfahren durchgeführt werden, wie die von J. Sambrook et al. in Molecular Cloning, A Laboratory Manual, 2nd Ed., Cold Spring Harbor Laboratory (1989) beschriebenen. Unter stringenter Bedingung ist eine Bedingung gemeint, bei der das Waschen bei 65 °C und einer Salzkonzentration von 0,1x bis 2x SSC durchgeführt wird (wobei unter 1xSSC eine Mischung aus 0,15 M Natriumchlorid/0,015 M Natriumcitrat zu verstehen ist).

### Materialien

D-Mannitol wurde von Sigma-Aldrich, D-Fructose, 2-Propanol, Acetonitril, NAD⁺, NADH-Dinatriumsalz, NADP⁺-Dinatriumsalz und NADPH-Tetranatriumsalz wurden von PanReac AppliChem (ITW Reagents), IPTG (Isopropyl-β-D-thiogalactopyranosid), Kaliumdihydrogenphosphat, di-Kaliumhydrogenphosphat, sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F' transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optischen Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA)-HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymklassen und Spenderorganismen für die in den Beispielen verwendeten Enzyme.**

| **Enzymtyp (EC-Klasse)** | **katalysierte Reaktion** | **Spenderorganismus** | **Literatur/SEQ ID Nr.** |
|---|---|---|---|
| Mannitol-Dehydrogenase I (MDH I) (EC 1.1.1.67) | D-Fructose → D-Mannitol | *Gluconobacter oxydans* DSM 3504 | (NCBI Protein Database: WP_041111406.1); SEQ ID Nr. 2 |
| Mannitol-Dehydrogenase II (MDH II) (EC 1.1.1.138) | D-Fructose → D-Mannitol | *Debaryomyces fabryi* | (NCBI Protein Database: XP_015465740.1)*; SEQ ID Nr. 4 |
| Alkoholdehydrogenase I (ADH I) (EC 1.1.1.1) | 2-Propanol → Aceton | *(Geo-)Bacillus stearothermophilus* NCA1503 | (Sakoda & Imanaka, 1992) |
| Alkoholdehydrogenase II (ADH II) (EC 1.1.1.2) | 2-Propanol → Aceton | *Thermoanaerobacter pseudethanolicus* | (NCBI Protein Database: ABY93890.1) |

| | | | |
|---|---|---|---|
| * Anmerkung: Im NCBI-Datenbankeintrag ist die Mannitol-Dehydrogenase II als Sorbose-Reduktase klassifiziert, allerdings wird die Region 27 bis 276 im Eintrag auch als "mannitol dehydrogenase (MDH)-like, classical (c) SDRs; cd05352" beschrieben. Die MDH-Aktivität von Sorbose-Reduktasen ist auch aus der Literatur bekannt (z.B. Sugisawa et al., 1991). | | | |

### Analytische Methoden

### High Performance Liquid Chromatography

Zur Quantifizierung von D-Fructose und D-Mannitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Ca2+ (8%) Säule mit entsprechender Vorsäule verwendet und mit 3,5 v% 2-Propanol isokratisch eluiert (Flussrate 0,5 ml/min).

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67.10⁻⁸ kat).

Mit dem nachfolgenden Beispiel wird eine bevorzugte Variante des erfindungsgemäßen Verfahrens noch näher beschrieben. Die in diesem Beispiel eingesetzten Lysate wurden nach den oben beschriebenen Verfahren hergestellt.

### Beispiel

### Reduktion von D-Fructose zu D-Mannitol

In einem 2 ml Glas-Vial (Ansatz 1) wurden folgende Komponenten vermischt: 211 µl deionisiertes Wasser, 50 µl eines 500 mM TEA-HCl-Puffers (pH 8), 125 µl einer 600 g/l D-Fructose-Lösung (finale Konzentration 150 g/l) sowie 10 µl einer 5 mM NAD⁺-Lösung (finale Konzentration 0,1 mM). Zum Start der Reaktion wurden 30 U MDH I-Lysat (SEQ ID Nr. 2) sowie 26 U ADH I-Lysat zum Ansatz hinzugefügt. Der Ansatz wurde unter kontinuierlichem Schütteln (30 °C, 1200 rpm) für insgesamt 45 h inkubiert.

In einem 2 ml Glas-Vial (Ansatz 2) wurden folgende Komponenten vermischt: 176 µl deionisiertes Wasser, 50 µl eines 500 mM TEA-HCl-Puffers (pH 8), 125 µl einer 600 g/l D-Fructose-Lösung (finale Konzentration 150 g/l) sowie 10 µl einer 2,5 mM NADP⁺-Lösung (finale Konzentration 0,05 mM). Zum Start der Reaktion wurden 30 U MDH II-Lysat (SEQ ID Nr. 4) sowie 29 U ADH II-Lysat zum Ansatz hinzugefügt. Der Ansatz wurde unter kontinuierlichem Schütteln (30 °C, 1200 rpm) für insgesamt 45 h inkubiert.

Bei beiden Ansätzen wurden nach 15,5 h 20 µl 2-Propanol und 30 µl deionisiertes Wasser, nach 24 h 15 µl 2-Propanol, nach 39,5 h 20 µl 2-Propanol und 30 µl deionisiertes Wasser sowie nach 43,5 h 8 µl 2-Propanol nachdosiert, um die Verluste durch Verdunstung (2-Propanol und Wasser) und Umsetzung (2-Propanol zu Aceton) auszugleichen.

Zur Analyse wurden 50 µl eines Ansatzes mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial überführt und mittels HPLC (RI-Detektion vermessen).

Auf diese Weise konnte die D-Fructose (150 g/l) in beiden Ansätzen vollständig zu D-Mannitol umgesetzt werden.

### Literatur

Saha, B. C., & Racine, F. M. (2011). Biotechnological production of mannitol and its applications. Applied Microbiology and Biotechnology, 89, 879-891. https://doi.org/10.1007/s00253-010-2979-3
Bhatt, S. M., Mohan, A., & Srivastava S. K. (2013). Challenges in enzymatic route of mannitol production. ISRN Biotechnology, 2013, 914187. https://doi.org/10.5402/2013/914187
Chen, M., Zhang, W., Wu, H., Guang, C., & Mu, W. (2020). Mannitol: physiological functionalities, determination methods, biotechnological production, and applications. Applied Microbiology and Biotechnology, 104, 6941-6951. https://doi.org/10.1007/s00253-020-10757-y
Martínez-Miranda, J. G., Chairez, I., & Durán-Páramo, E. (2022). Mannitol Production by Heterofermentative Lactic Acid Bacteria: a Review. Applied Biochemistry and Biotechnology, 194, 2762-2795. https://doi.org/10.1007/s12010-022-03836-5
Zhang, M., Gu, L., Cheng, C., Zhu, J., Wu, H., Ma, J., Dong, W., Kong, X., Jiang, M., & Ouyang, P. (2017). High-yield production of mannitol by Leuconostoc pseudomesenteroides CTCC G123 from chicoryderived inulin hydrolysate. Journal of Industrial Microbiology and Biotechnology, 44(8), 1237-1244. https://doi.org/10.1007/s10295-017-1953-9
Kavanagh, K. L., Klimacek, M., Nidetzky, B., & Wilson, D. K. (2002). Crystal structure of Pseudomonas fluorescens mannitol 2-dehydrogenase binary and ternary complexes. Specificity and catalytic mechanism. Journal of Biological Chemistry, 277(45), 43433-43442. https://doi.org/10.1074/jbc.M206914200
Krahulec, S., Armao, G. C., Klimacek, M., & Nidetzky, B. (2011). Enzymes of mannitol metabolism in the human pathogenic fungus Aspergillus fumigatus - kinetic properties of mannitol-1-phosphate 5-dehydrogenase and mannitol 2-dehydrogenase, and their physiological implications. FEBS Journal, 278(8), 1264-1276. https://doi.org/10.1111/j.1742-4658.2011.08047.x
Kulbe, K. D., Schwab, U., & Gudernatsch, W. (1987). Enzyme-Catalyzed Production of Mannitol and Gluconic Acid - Product Recovery by Various Procedures. Annals of the New York Academy of Sciences, 506(1), 552-568. https://doi.org/10.1111/j.1749-6632.1987.tb23850.x
Slatner, M., Nagl, G., Haltrich, D., Kulbe, K. D., & Nidetzky, B. (1998). Enzymatic Production of Pure D-Mannitol at High Productivity. Biocatalysis and Biotransformation, 16(5), 351-363. https://doi.org/10.3109/10242429809003628
Xu, W., Lu, F., Wu, H., Zhang, W., & Guang, C. (2020). Identification of a highly thermostable mannitol 2-dehydrogenase from Caldicellulosiruptor morganii Rt8.B8 and its application for the preparation of D-mannitol. Process Biochemistry, 96, 194-201. https://doi.org/10.1016/j.procbio.2020.05.014
Neuhauser, W., Steininger, M., Haltrich, D., Kulbe, K. D., & Nidetzky, B. (1998). A pH-Controlled Fed-Batch Process Can Overcome Inhibition by Formate in NADH-Dependent Enzymatic Reductions Using Formate Dehydrogenase-Catalyzed Coenzyme Regeneration. Biotechnology and Bioengineering, 60(3), 277-282. https://doi.org/10.1002/(SICI)1097-0290(19981105)60:3<277::AID-BIT2>3.0.CO;2-E
Kratzer, R., Woodley, J. M., & Nidetzky, B. (2015). Rules for biocatalyst and reaction engineering to implement effective, NAD(P)H-dependent, whole cell bioreductions. Biotechnology Advances, 33(8), 1641-1652. https://doi.org/10.1016/j.biotechadv.2015.08.006
Boldt, A., & Ansorge-Schumacher, M. B. (2020). Formate Dehydrogenase from Rhodococcus jostii (RjFDH) - A High-Performance Tool for NADH Regeneration. Advanced Synthesis und Catalysis, 362(19), 4109-4118. https://doi.org/10.1002/adsc.202000536
Tishkov, V. I., & Popov, V. O. (2004). Catalytic Mechanism and Application of Formate Dehydrogenase. Biochemistry (Moscow), 69(11), 1252-1267. https://doi.org/10.1007/s10541-005-0071-x
Kulbe, K. D., Schwab, U., Howaldt, M., & Kimmerle, K. (1986). ANWENDUNG VON MEMBRANVERFAHREN BEI DER SIMULTANEN HERSTELLUNG VON MANNITOL UND GLUCONSAURE AUS SACCHAROSE DURCH KONJUGIERTE NAD+-ABHÄNGIGE DEHYDROGENASEN. Technische Membranen in der Biotechnologie, 189-200. http://hdl.handle.net/10033/623390
Lee, J.-K., Koo, B.-S., Kim, S.-Y., & Hyun, H.-H. (2003). Purification and characterization of a novel mannitol dehydrogenase from a newly isolated strain of Candida magnoliae. Applied Environmental Microbiology, 69(8), 4438-4447. https://doi.org/10.1128/AEM.69.8.4438-4447.2003
Ceccaroli, P., Saltarelli, R., Guescini, M., Polidori, E., Buffalini, M., Menotta, M., Pierleoni, R., Barbieri, E., & Stocchi, V. (2007). Identification and characterization of the Tuber borchii D-mannitol dehydrogenase which defines a new subfamily within the polyol-specific medium chain dehydrogenases. Fungal Genetics and Biology, 44(10), 965-978. https://doi.org/10.1016/j.fgb.2007.01.002
Soetaert, W., Vanhooren, P. T., Vandamme, E. J. (1999). The Production of Mannitol by Fermentation. In: Bucke, C. (eds) Carbohydrate Biotechnology Protocols. Methods in Biotechnology™, vol 10. Humana Press. https://doi.org/10.1007/978-1-59259-261-6_21
Cosse, K., Sharma, V., Allgeier, A., & McFarlane, J. (2021). Pseudomonas fluorescens Mannitol-2-Dehydrogenase as an NAD+ Recycling Enzyme in the Conversion of Ethanol to Acetaldehyde. The FASEB Journal, 35(S1). https://doi.org/10.1096/fasebj.2021.35.S1.01896
Cosse, K. J., Sharma, V. K., Allgeier, A. M., & McFarlane, J. S. (2022). Enzymatic production of acetaldehyde using a Pseudomonas fluorescens mannitol-2-dehydrogenase mutant to regenerate NAD+. The FASEB Journal, 36(S2). https://doi.org/10.1096/fasebj.2022.36.S1.R2882
Lee, J.-K., Song, J.-Y., & Kim, S.-Y. (2008). Controlling Substrate Concentration in Fed-Batch Candida magnoliae Culture Increases Mannitol Production. Biotechnology Progress, 19(3), 768-775. https://doi.org/10.1021/bp034025o
Xu, J., Zhou, H., Yu, H., Deng, T., Wang, Z., Zhang, H., Wu, J., & Yang, L. (2021). Computational design of highly stable and soluble alcohol dehydrogenase for NADPH regeneration. Bioresources and Bioprocessing, 8, 12. https://doi.org/10.1186/s40643-021-00362-w
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. WP_041111406.1, mannitol dehydrogenase family protein [Gluconobacter oxydans]. Verfügbar unter: https://www.ncbi.nlm.nih.gov/protein/WP_041111406.1
Protein [Internet]. Bethesda (MD): National Library of Medicine (US), National Center for Biotechnology Information. Accession No. XP_015465740.1, Sorbose reductase SOU1 [Debaryomyces fabryi]. Verfügbar unter: https://www.ncbi.nlm.nih.gov/protein/XP_015465740.1
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Protein [Internet]. Bethesda (MD): National Library of Medicine (US), National Center for Biotechnology Information. Accession No. ABY93890.1, Alcohol dehydrogenase, zinc-binding domain protein [Thermoanaerobacter pseudethanolicus ATCC 33223]. Verfügbar unter: https://www.ncbi.nlm.nih.gov/protein/ABY93890.1
Sugisawa, T., Hoshino, T., & Fujiwara, A. (1991). Purification and Properties of NADPH-Linked L-Sorbose Reductase from Gluconobacter melanogenus N44-1. Agricultural and Biological Chemistry, 55(8), 2043-2049. https://doi.org/10.1080/00021369.1991.10870899

## Patentansprüche

1. Verfahren zur Herstellung einer wässerigen Lösung enthaltend D-Mannitol, indem D-Fructose, welche in einer wässerigen Lösung gelöst vorliegt, mit einer NAD(P)H-abhängigen Oxidoreduktase unter Bildung von oxidiertem Kofaktor NAD(P)⁺ zu D-Mannitol reduziert wird, **dadurch gekennzeichnet, dass** der durch die Reduktion entstandene, oxidierte Kofaktor NAD(P)⁺ mit einer Alkoholdehydrogenase und einem sekundären Alkohol unter Bildung eines Ketons zu NAD(P)H reduziert wird.

2. Verfahren nach Anspruch 1, **dadurch gekennzeichnet, dass** der sekundäre Alkohol 2-Propanol ist.

3. Verfahren nach einem der Ansprüche 1 oder 2, **dadurch gekennzeichnet, dass** die Reduktion von D-Fructose zu D-Mannitol *in vitro* durchgeführt wird.

4. Verfahren nach Anspruch 3, **dadurch gekennzeichnet, dass** die Oxidoreduktase als Lysat der entsprechenden, sie bildenden Zellen vorliegt.

5. Verfahren nach einem der Ansprüche 1 bis 4, **dadurch gekennzeichnet, dass** die NAD(P)H-abhängige Oxidoreduktase zur Reduktion von D-Fructose zu D-Mannitol eine Mannitol-Dehydrogenase ist und vorzugsweise eine Aminosäuresequenz umfasst, welche ausgewählt ist aus der Gruppe bestehend aus:
i) einer Aminosäuresequenz, die eine Identität zu SEQ ID Nr. 2 oder 4 von mindestens 80% aufweist,
ii) einer Aminosäuresequenz, die durch eine Nukleinsäure kodiert wird, die eine Identität zu SEQ ID Nr. 1 oder 3 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 1 oder 3 bindet.
